# EUROPÄISCHE PATENTSCHRIFT

(11) **EP 2 114 375 B1**
(45) Veröffentlichungstag und Bekanntmachung des Hinweises auf die Patenterteilung: **30.11.2011**
(21) Anmeldenummer: 07857369.8
(22) Anmeldetag: 11.12.2007
(51) Int. Cl.: A61K 9/28, A61K 9/14

(54) **SCHNELLDISPERGIERBARES, TEILCHENFÖRMIGES FILMÜBERZUGSMITTEL BASIEREND AUF POLYVINYLALKOHOL-POLYETHER-PFROPFCOPOLYMEREN**
RAPIDLY DISPERSABLE, PARTICULATE FILM COATING AGENT BASED ON POLYVINYL ALCOHOL-POLYETHER GRAFT COPOLYMERS
AGENT D'ENROBAGE SOUS FORME DE FILM, FORMÉ DE PARTICULES À DISPERSION RAPIDE, À BASE DE COPOLYMÈRES GREFFÉS DE POLYVINYLALCOOL-POLYÉTHER

(30) Priorität: 29.12.2006 EP 06127337
(43) Veröffentlichungstag der Anmeldung: 11.11.2009
(73) Patentinhaber: BASF SE, 67056 Ludwigshafen (DE)
(72) Erfinder: KOLTER, Karl, 67117 Limburgerhof (DE); MASCHKE, Angelika, 93047 Regensburg (DE); DIETZEN, Franz-Josef, 67454 Hassloch (DE); SCHMELLER, Thorsten, 67157 Wachenheim (DE)
(86) Internationale Anmeldenummer: PCT/EP2007/063674
(87) Internationale Veröffentlichungsnummer: WO 2008/080774

(56) Entgegenhaltungen:
- EP-A- 1 136 254
- EP-A- 1 674 485
- WO-A-00/18375
- WO-A-03/070224
- WO-A-2006/002808
- WO-A-2006/018130
- US-A1- 2005 196 444

## Beschreibung

Die vorliegende Erfindung betrifft feste schnell dispergierbare Filmüberzugsmittel zum Überziehen von pharmazeutischen Darreichungsformen oder Nahrungsergänzungsmitteln, die aus mindestens einem Polyvinylalkohol-Polyether-Pfropfcopolymer (Komponente A) und weiteren üblichen Coatingbestandteilen, insbesondere farbgebenden Mitteln, bestehen. Ferner betrifft die Erfindung ein Verfahren zur Herstellung von solchen festen teilchenförmigen Filmüberzugsmitteln.

Feste Darreichungsformen werden aus den verschiedensten Gründen mit einem schnelllöslichen Überzug versehen. So kann beispielsweise das Aussehen, die Unterscheidbarkeit und die Schluckbarkeit verbessert werden, ein bitterer Geschmack überdeckt werden oder die Darreichungsform gegenüber äußeren Einflüssen wie z. B. Feuchtigkeit oder Sauerstoff geschützt werden. Da sich im Falle von schnell freisetzenden Darreichungsformen der Filmüberzug schnell in verschiedenen wässrigen Medien wie in künstlichem Magen- und Darmsaft lösen soll, muss der wichtigste Bestandteil des Filmüberzugsmittels ein wasserlösliches, filmbildendes Polymer sein. Zum Überziehen von Tabletten werden als filmbildende Polymere hauptsächlich Hydroxypropylmethylcellulose und Hydroxypropylcellulose eingesetzt, die jedoch gravierende Nachteile aufweisen. So ist die Viskosität dieser Polymere in Wasser sehr hoch und erlaubt nur eine Konzentration bis ca. 10 %, da aufgrund der hohen Viskosität bei höheren Konzentrationen keine feine Zerstäubung in der Sprühdüse mehr möglich ist und der Überzug rau, inhomogen und unansehnlich wird. Weiterhin sind diese Polymere sehr spröde und erleiden häufig Risse während der Lagerung, insbesondere wenn der Kern durch Feuchtigkeitsaufnahme oder -abgabe sein Volumen verändert.

Die Verwendung von Polyvinylalkohol-Polyether-Pfropfcopolymeren als Überzugsmittel oder Bindemittel in pharmazeutischen Darreichungsformen oder als Verpackungsmaterial oder als Zusatzstoff in kosmetischen, dermatologischen oder hygienischen Zubereitungen ist beispielsweise aus der WO 00/18375 bekannt. So wird beispielsweise eine Rezeptur für ein Filmüberzugsmittel beschrieben, welches aus einem Polyvinylalkohol-Polyether-Pfropfcopolymer und den üblichen Coatingbestandteilen zum Färben und Decken, nämlich Eisenoxid, Talkum und Titandioxid besteht. Jedoch ließen die dort beschriebenen Mischungen noch Raum für Verbesserungen.

In der WO 03/070224 werden Überzüge beschrieben, die aus Polyvinylalkohol-Polyether-Pfropfcopolymeren, einer Komponente mit Hydroxy-, Amid- oder Esterfunktionen und weiteren üblichen Coatingbestandteilen bestehen. Dabei wird zunächst ein Prämix der Einsatzstoffe als physikalische Mischung hergestellt und diese anschließend in Wasser dispergiert. Diese Zubereitungen neigen zur Entmischung und weisen keine guten Rauhigkeitswerte auf.

Andererseits besteht aber ein großes Interesse an fertigen Vormischungen für FilmÜberzugsmittel, die neben dem filmbildenden Polymer übliche Hilfsstoffe wie beispielsweise Pigmente oder weitere Inhaltsstoffe enthalten, die sich nicht entmischen, was für die gleichbleibende Qualität des Überzugs von Bedeutung ist, nicht stauben und gut lagerbar sind und vor der Anwendung als Filmüberzugsmittel einfach und schnell redispergiert werden können.

Aus der WO 06/002808 sind pulverförmige Filmüberzugsmittel, enthaltend neben Polyvinylalkohol-Polyether-Pfropfpolymeren Polyvinylpyrrolidone, Pigmente und Tenside, bekannt, die durch Mahlung der Pigmente in Gegenwart von wässrigen Lösungen des Polymeren und der anderen Inhaltsstoffe und anschließende Sprühtrocknung erhalten werden. Diese Filmüberzugsmitttel lassen jedoch im Hinblick auf die Staubfreiheit und Handhabbarkeit für den Anwender noch Raum für Verbesserungen

Der Erfindung lag die Aufgabe zugrunde, einen Filmüberzug zu entwickeln, der in fester Form zu keinerlei Entmischung zwischen den einzelnen Bestandteilen führt, insbesondere nicht zwischen Pigmenten und Polymeren, der ausgezeichnet fließfähig ist, der sehr einfach und schnell in Wasser aufzulösen ist, wodurch sich eine sehr kurze Herstellungszeit der Sprühzubereitung ergibt, der in hohen Polymer- und Feststoffkonzentrationen und mit hoher Sprührate zu versprühen ist, ohne dass die Sprühdüse verblockt, der sehr gut auf der Oberfläche spreitet, der flexibel ist und keinerlei Rissbildung während der Lagerung aufweist, der nicht klebrig ist, der auf allen Oberflächen gut haftet, der ausgezeichnete Glätte und Glanz aufweist, der gegenüber mechanischer Belastung sehr stabil ist und sich sehr schnell auflöst. Insbesondere lag die Aufgabe darin, feste Filmüberzugsmittel zu finden, die hinsichtlich Staubfreiheit und elektrostatischer Aufladung gut handhabbar sind.

Demgemäß wurden feste teilchenförmige Filmüberzugsmittel, bestehend aus
a) 40 - 90 Gew.-%, bevorzugt 40 bis 70 Gew.-% eines Polyvinylalkohol-Polyether-Pfropfcopolymeren (Komponente A),
b) 10 bis 60 Gew.-%, bevorzugt 20 bis 45 Gew.-% eines bunten oder unbunten farbgebenden Mittels(Komponente B)
c) 0 bis 30 Gew.-% weiteren üblichen Hilfsstoffen (Komponenten C)
gefunden, wobei sich die Menge der Komponenten A bis C zu 100 Gew.-% addiert, die durch Extrusion einer plastischen Masse der Komponenten A) bis C) und anschliessende Formgebung erhalten werden.

Die Filmüberzugsmittel weisen eine enge Teilchengrößenverteilung auf, wobei die mittlerenTeilchengrößen (d05,Volumenmittel) im Bereich von 300 µm bis 2000 µm, vorzugsweise 500 µm bis 1500 µm liegen. Die Verteilungsbreite beträgt <1, vorzugsweise <0,8. Die Verteilungsbreite berechnet sich nach folgender Formel: (d(09)-d(01))/ d(05). Die erfindungsgemäßen Filmüberzugsmittel bestehen aus ovalen bis rundlichen linsenförmigen Teilchen.

Die Filmüberzugsmittel sind im Anwendungsmedium, insbesondere in Wasser, schnell dispergierbar.

Die Filmüberzugsmittel werden nach einem Verfahren erhalten, welches dadurch gekennzeichnet ist, dass die Komponenten A) und B) und gegebenenfalls C) zu einer plastischen Masse verarbeitet, extrudiert und der Formgebung unterworfen werden.

Unter Polyvinylalkohol-Polyether-Pfropfcopolymeren werden Polymere verstanden, die erhältlich sind durch Polymerisation von
a) mindestens einem Vinylester von aliphatischen C₁-C₂₄-Carbonsäuren bevorzugt Vinylacetat, in Gegenwart von
b) Polyethern der allgemeinen Formel I,

   R¹⁻O-(R²⁻O)ₓ₋(R³⁻O)_{y-}(R⁴⁻O)_{z-}R⁵ I
c) in der die Variablen unabhängig voneinander folgende Bedeutung haben:
   - R¹: Wasserstoff, C₁-C₂₄-Alkyl, R⁶⁻C(=O)-, Polyalkoholrest;
   bevorzugt: R₁=H, CH₃-
   - R⁵: Wasserstoff, C₁-C₂₄₋Alkyl, R⁶⁻C(=O)-;
   bevorzugt: R⁵=H
   - R² bis R⁴: -(CH₂)₂₋, -(CH₂)₃₋,-(CH₂)₄₋, -CH₂-CH(CH₃)-,
   -CH₂-CH(CH₂-CH₃)-, -CH₂₋CHOR⁷⁻CH₂₋;
   bevorzugt R₂ bis R₄: .(CH₂)₂₋, -CH₂₋CH(CH₃)-
   ganz besonders bevorzugt R₂ bis R₄: -(CH₂)₂-
   - R₆: C₁-C₂₄₋Alkyl;
   - R₇: Wasserstoff, C₁-C₂₄₋Alkyl, R⁶⁻C(=O)-;
   - x: 1 bis 5000;
   bevorzugt: x = 10 bis 2000;
   ganz besonders bevorzugt: x = 20 bis 500
   - y: 0 bis 5000;
   bevorzugt: y = 0
   - z: 0 bis 5000;
   bevorzugt: z = 0,
   mit der Maßgabe, dass x ≥ 10 ist, wenn y und z = 0 sind,
   und anschließende vollständige oder teilweise Verseifung der Polyvinylestergruppen.
   x, y, z:
   die Berechnung des Molekulargewichts des Polyethers aus x, y, z erfolgt als Mittelwert, da entsprechende Produkte meist eine breite Molgewichtsverteilung besitzen.

Bevorzugt sind Polyether mit einem mittleren Molgewicht zwischen 400 und 50000 g/mol, besonders bevorzugt 1500 bis 20000 g/mol.

Die Herstellung solcher Pfropfcopolymere ist an sich bekannt und zum Beispiel in der WO 00/18357, auf die hiermit Bezug genommen wird, beschrieben.

Bevorzugt sind Polymere mit einem Verseifungsgrad der Polyvinylestergruppen von > 70 mol-%, besonders bevorzugt > 80 mol-% und ganz besonders bevorzugt von > 85 mol-%.

Besonders bevorzugt ist ein Polyvinylalkohol-Polyether-Pfropfcopolymer bei dem
a) Vinylacetat als zu pfropfendes Monomer verwendet wurde,
b) die Variablen folgende Bedeutung haben:
   - R¹ =: H
   - R² - R⁴ =: -(CH₂)₂-
   - R₅=: H
   - x =: 20 bis 500
   - y =: 0
   - z =: 0
   und somit ein Polyethylenglykol mit einem mittleren Molekulargewicht von 6000 repräsentieren
c) der Verseifungsgrad der Estergruppen > 85 mol-% liegt und
d) das Massenverhältnis der Molekülteile Polyvinylalkohol / Polyethylenglykol 6000 bei 75:25 liegt.

Weiterhin enthalten die Filmüberzugsmittel als Komponenten B farbgebende Mittel, insbesondere organische oder anorganische Pigmente. Als farbgebende Mittel können bunte oder unbunte farbgebende Mittel eingesetzt werden, wobei erfindungsgemäß unter unbunten Mitteln weiße, graue oder schwarze Mittel, bevorzugt weiße Mittel, insbesondere Weißpigmente, verstanden werden.

Als Pigmente werden farbgebende oder weiße Substanzen bezeichnet, die im Anwendungsmedium nicht löslich sind.

Die Pigmente können dem erfindungsgemäßen Verfahren in reiner Form oder als Pigmentpräparationen zugeführt werden. Solche Pigmentpräparationen sind dem Fachmann bekannt und ebenso wie die reinen Pigmente kommerziell erhältlich.

Als anorganische Pigmente eignen sich Aluminiumsilikate, Magnesiumsilikate, Magnesium-aluminiumsilikate, Eisenoxid, Titandioxid, Zinkoxid, Kieselsäure, oder Calciumhydrogenphosphat. Von den Aluminiumsilikaten kommt vor allem Kaolin in Betracht. Von den Magnesiumsilikaten hat vor allem Talkum Bedeutung.
Bevorzugte Pigmente sind Eisenoxid oder Eisenoxid-Präparationen, beispielsweise die kommerziell erhältlichen Sicovit® Gelb 10 E172 oder Sicovit® Rot 30 E172, und Weißpigmente ausgewählt aus der Gruppe bestehend aus Titandioxid, Talkum und Kaolin.

Als organische Pigmente eignen sich organische Farblacke oder Mischungen davon. Als organische Farblacke können z.B. verwendet werden: Carminlack,Chinolingelblack, Tartrazinlack, Gelborangelack, FD&C Gelb-Aluminiumlack, Cochenillerotlack, Erythrosinlack, Azorubinlack, Indigotinlack, Brilliantblau, Betacarotin.

Weiterhin können die Filmüberzüge als Komponenten C gegebenenfalls bis zu 30 Gew.-% an Hilfsstoffe enthalten, wie sie als Coatingbestandteile üblich sind. Weitere übliche Coatingbestandteile umfassen:
Tenside, wasserlösliche Farbstoffe, Antiklebemittel, polymere Bindemittel, Füllstoffe, Quellmittel, Glanzverstärker, Schaumverhinderer, Schutzkolloide, Puffersubstanzen, pH-regulierenden Substanzen, Haftvermittler oder Weichmacher.

Als Tenside können Substanzen mit einem HLB-Wert (Hydrophilic Lipophilic Balance; Vgl. Fiedler, Lexikon der Hilfsstoffe, Editio Cantor Verlag Aulendorf, 5.Auflage (2002), Seite 115-121) größer 10 eingesetzt werden.

Besonders geeignet sind Alkalisalze von C8-C30-Fettsäuren , C8-C30-.Alkylsulfonaten, C8-C30-Alkylsulfaten, C8-C30-Alkylarylsulfonaten oder Dioctylsulfosuccinat, Ethoxylate von C8-C30-Fettsäuren, C8-C30-Fettalkoholen, Fettsäureglyceriden, Sorbitanfettsäureestern, Sorbitanfettalkoholethern oder Phenolen, sowie Polyoxypropylen-Polyoxyethylen-Blockcopolymeren. Beispiele aus den genannten Stoffklassen sind Natriumstearat, Natriumoleat, Natriumlaurylsulfonat, Natriumlaurylsulfat, Polyoxyethylen(9)monostearat, Polyoxyethylen(10)stearylcetylether, Polysorbat 80, Polysorbat 20, ethoxyliertes Ricinusöl (35 EO), ethoxyliertes hydriertes Ricinusöl (40 EO), ethoxylierte 12-Hydroxystearinsäure (15 EO), Poloxamer 188, Poloxamer 408.

Als Füllstoffe eignen sich z.B. Cellulosen wie mikrokristalline Cellulose, weiterhin Zucker wie Lactose, Saccharose oder Dextrose, Zuckeralkohole wie Mannitol., Sorbitol, Xylitol oder Isomalt.

Als Quellmittel eignen sich Crospovidone, Croscarmellose, Natriumcarboxymethylstärke oder nicht vernetzte Carboxymethylcellulose.

Als polymere Bindemittel eignen sich Homo- und Copolymere von N-Vinylpyrrolidone, beispielsweise Povidon mit K-Werten nach Fikentscher von 12 bis 90 oder Copovidon.

Zur Herstellung der festen Dosierungsformen wird ein plastisches Gemisch der Komponenten bereitgestellt, das anschließend einem Formgebungsschritt unterzogen wird. Das Vermischen der Komponenten und die Plastifzierung des Gemisches können auf unterschiedliche Weise erfolgen.

Plastifzierung bedeutet ein Weichmachen der Mischung durch Einwirken von Druck, Scherkräften, Temperatur und/oder Weichmachern. Vorzugsweise erfolgt das Weichmachen im Sinne der Erzielung einer Thermoplastizität durch kombiniertes Einwirken von Temperaturerhöhung und Scherkräften, insbesondere in Kombination mit Weichmachern.

Als Weichmacher eignen sich langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester, insbesondere Triethylcitrat, Fettsäureester; Glycerinmono-, Glycerindi- oder Glycerintriacetat oder Natriumdiethylsulfosuccinat oder Mischungen der genannten Weichmacher. Weiterhin eignet sich als Weichmacher bevorzugt Wasser.
Die Menge an Weichmacher wird so bemessen, dass die Masse noch plastisch und extrudierbar bleibt. Üblicherweise wird die zugesetzte Menge 30 Gew.-%, bezogen auf die Summe der Menge an Komponenten A bis C nicht überschreiten.

Weiterhin können während der Verarbeitung auch porenbildende Mittel eingesetzt werden. Solche Mittel können zur Aufschäumung bei der Extrusion führen oder durch Porenbildung während der Auflösung des fertigen Überzugs zur Zerfallsbeschleunigung führen. Als solche Porenbildner eignen sich Carbonate oder Hydrogencarbonate von Alkali- oder Erdalkalimetallen wie beispielsweise Natrium, Kalium, Magnesium oder Calcium oder die entsprechenden Ammoniumverbindungen. Es kann auch gasförmiges Kohlendioxid eingesetzt werden. Weiterhin können auch Säuren, insbesondere organische Säuren wie Zitronensäure, Weinsäure, Milchsäure, Maleinsäure, Fumarsäure oder Bernsteinsäure zugesetzt werden.

Das Vermischen der Komponenten kann vor, während und/oder nach der Bildung der plastischen Masse erfolgen. Beispielsweise können die Komponenten zuerst vermischt und dann plastifziert oder gleichzeitig vermischt und dann plastifiziert werden. Häufig erfolgt noch eine Homogenisierung des plastischen Gemisches, um eine hochdisperse Verteilung des farbgebenden Mittels zu erhalten.

Im Allgemeinen werden die Komponenten als solche in das Herstellungsverfahren eingesetzt. Sie können jedoch auch in flüssiger Form, d.h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂. Die Lösungsmittel können gleichzeitig auch als Weichmacher dienen.

Welches Lösungsmittel im Einzelfall verwendet wird, hängt von der zu verarbeitenden Zusammensetzung ab. Auf jeden Fall ist die Menge an Lösungsmittel so zu begrenzen, dass die zu verarbeitende Masse plastisch und extrudierbar bleibt.

Das Plastifzieren und/oder Vermischen erfolgt in einer für diesen Zweck üblichen Vorrichtung. Besonders geeignet sind Extruder oder gegebenenfalls beheizbare Behälter mit Rührwerk, z.B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drücken von Vorteil ist, zugespeist werden. Flüssige Medien können über ein geeignetes Pumpenaggregat zudosiert werden.

Das durch Vermischen und/oder Erweichen des Filmüberzugsmittels, und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten.
Die Verfahrensschritte Vermischen und Plastifizieren können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend gegebenenfalls unter Zusatz weiterer Komponenten extrudiert werden.

Das erfindungsgemäße Verfahren erlaubt es, als Extruder Einschneckenmaschinen, kämmende Schneckenmaschinen oder auch Mehrwellenextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Zweischneckenextruder.

Das erfindungsgemäße Verfahren wird üblicherweise durch Plastifzieren bei erhöhter Temperatur durchgeführt, vorzugsweise bei Temperaturen von 50 bis 160 (Temperatur der plastischen Mischung), besonders bevorzugt 75 bis 120°C.

Die Düsenauslegung erfolgt dabei in Abhängigkeit von dem zur Anwendung kommenden polymeren Bindemittel und der gewünschten pharmazeutischen Form.

Das plastische Gemisch wird in der Regel einer abschließenden Formgebung unterzogen. Dabei kann eine Vielzahl von Formen, je nach Werkzeug und Art der Formung, erzeugt werden. Durch Extrusion und Heiß- oder Kaltabschlag des Stranges können weitere Formen erhalten werden, beispielsweise kleinteilige und gleichmäßig geformte Granulate. Die Heißgranulierung führt bevorzugt zu linsenförmigen Formen.

Aufgrund ihrer Staubfreiheit, ihrer gleichmässigen Teilchengrößenverteilung, ihrer hohen Schüttdichte, ihrer guten Fließfähigkeit und ihrer Form weisen die erfindungsgemäßen Filmüberzugsmittel besonders anwendungsfreundliche Eigenschaften auf. Sie sind nicht nur gut redispergierbar, sondern auch sonst gut handhabbar und vorteilhaft hinsichtlich der geringen elektrostatischen Aufladbarkeit.

Die Schüttdichte der erfindungsgemäßen Filmüberzugsmittel liegt bei Werten größer 0,4, vorzugsweise größer 0,5 und besonders bevorzugt größer 0,6 g/ml.

Die erfindungsgemäßen Filmüberzugsmittel eignen sich grundsätzlich als Filmüberzugsmittel für alle Dosierungsformen von biologisch aktiven Substanzen. Unter Dosierungsformen sind hier alle Formen zu verstehen, die zur Verwendung als Arzneimittel, Pflanzenbehandlungsmittel, Futtermittel und Nahrungsmittel und zur Abgabe von Riechstoffen und Parfümölen oder anderen kosmetischen Wirkstoffen geeignet sind. Dazu gehören beispielsweise Tabletten jeglicher Form, Pellets oder Granulate. Weiterhin können auch Hart- oder Weichkapseln überzogen werden

Solche Dosierungsformen umfassen im Allgemeinen:
I 0.1 bis 99 Gew.-%, insbesondere 0,1 bis 60 Gew.-% (bezogen auf das Gesamtgewicht der Dosierungsform) eines Wirkstoffes,
II 1 bis 99,9 Gew.-%, insbesondere 20 bis 99 Gew.-% eines Bindemittels oder Füllstoffs und
III 0 bis 30 Gew.-% weiterer Additive ,
wobei sich die Mengen von I, II and III zu 100 Gew.-% addieren

Als Bindemittel geeignet sind beispielsweise:
Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyvinylalkohol-Polyether-Pfropfcopolymere, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Weiterhin können die Dosierungsformem auch Weichmacher wie langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester, insbesondere Triethylcitrat), Fettsäureester, wie Glycerinmono-, Glycerindi- oder Glycerintriacetat oder Natriumdiethylsulfosuccinat oder Mischungen der genannten Weichmacher. Die Konzentration an Weichmacher beträgt im Allgemeinen 0,5 bis 15, vorzugsweise 0,5 bis 10 Gew.-%, bezogen auf das Gesamtgewicht des Gemisches, enthalten.

Übliche galenische Füllstoffe sind Silikate oder Kieselerde, Dicalciumphosphat, Calciumcarbonat, Magnesiumoxid, Aluminiumoxid, Talkum, Saccharose, Glucose, Lactose, Zuckeralkohole wie Sorbit, Mannit, Xylit, Maltit, Isomalt, Getreide- oder Maisstärke, Kartoffelstärke, mikrokristalline Cellulose.

Übliche Schmiermittel sind Aluminium- und Calciumstearat, Talkum und Silicone, und können in einer Konzentration von 0,1 bis 5, vorzugsweise 0,1 bis 3 Gew.-%, bezogen auf das Gesamtgewicht des Gemisches, enthalten sein, desgleichen auch tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab.

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Retardierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Die zu coatenden Darreichungsformen können auch Substanzen zur Herstellung einer festen Lösung des Wirkstoffs enthalten. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Die zu coatenden Darreichungsformen können durch Granulation, Kristallisation, Kompaktierung, Verpressung, Extrusion, Erstarrung oder Verkapselung hergestellt werden.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 0,5 bis 80, insbesondere 1 bis 70 Gew.-% liegen. Auch WirkstoffKombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Die erfindungsgemäßen Filmüberzugsmittel sind beispielsweise zur Beschichtung von Dosierungsformen folgender Wirkstoffe geeignet:
Acebutolol, Acetylcystein, Acetylsalicylsäure, Acyclovir, Alfacalcidol, Allantoin, Allopurinol, Alprazolam, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Trandolapril, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Zidovudin.

Erfindungsgemäß eignen sich die Filmüberzugsmittel auch für Dosierungsformen, die als mehrschichtige pharmazeutische Formen durch Koextrusion hergestellt werden können, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten. Auf diese Weise lassen sich mehrstufige Freisetzungsprofile einstellen.

Mehrschichtige Arzneiformen umfassen vorzugsweise zwei oder drei Schichten. Sie können in offener oder geschlossener Form vorliegen, insbesondere als offene oder geschlossene Mehrschichttabletten. Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann. Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet.

Im Einzelnen kann es zur Ausbildung von festen Lösungen kommen.
Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Überraschenderweise ließen die Pfropfpolymerisate sich nach dem erfindungsgemäßen Verfahren zu Filmüberzugsmitteln verarbeiten, ohne dass es zu Vernetzung kam. Der Fachmann hätte aufgrund der Struktur der Pfropfpolymerisate bei der Belastung durch Temperatur und Scherkräfte eine Vernetzung unter Wasserabspaltung erwartet.

Die folgenden Beispiele sollen die Erfindung veranschaulichen, ohne sie jedoch zu beschränken.

### Beispiele

### Allgemeine Vorschrift

Als Extruder wurde ein gleichsinnig drehender Zweischnecken-Extruder ZSK 25/1 der Firma Werner & Pfleiderer mit einem Schneckendurchmesser von 25 mm verwendet. Der mit einer Einzugsvorrichtung und einer Entlüftungsvorrichtung und versehene Extruder wurde mit 8 unterschiedlichen Temperaturzonen betrieben. Die Mischung aus Pfropfpolymer und Komponenten C wurde in den ersten drei Zonen bei 85-90°C intensiv gemischt und geknetet, in Zone 4 gefördert und in Zone 5 mit den Pigmenten vermischt. Anschliessend wurde die Masse in den Zonen 6 bis 8 bei 100°C oder bei 110°C gefördert und anschließend extrudiert.

Die plastifizierte Masse wurde über Loch-Düsenplatten extrudiert. Verwendet wurden Düsenplatten mit einem Lochdurchmesser von 1 mm oder 0.8 mm. Die Schneckengeschwindigkeit betrug von 100 bis 300 Upm bei einem Durchsatz von 1.5 bis 3 kg/h. Die austretenden Stränge wurden durch Abschlag mit einem rotierenden Messer geformt und die entstehenden Teilchen bei 25 -40°C für 12 h in einem Vakuumtrockenschrank getrocknet.

Als Pfropfpolymerisat wurde ein Polymerisat aus 75 Gew.-% Polyvinylalkohol-Einheiten und 25 Gew.-% Polyethylenglykol als Pfropfgrundlage (PEG 6000) mit einem Molekulargewicht im Bereich von 45.000 Dalton und einem verseifungsgrad von 94 mol-% eingesetzt. Alle eingesetzten Sicovit®-Typen (Eisenoxide: Sicovit Gelb 10E172, Sicovit Rot 30 E172; Sicovit Indigotinlack) sind kommerziell erhältlich (Firma BASF Aktiengesellschaft).

Auf diese Weise wurden Formulierungen der nachstehend aufgeführten Zusammensetzungen verarbeitet. Die Angaben in % beziehen sich auf Gew.-%.

| Formulierung Nr. | 1 | 2 | 3 | 4 |
|---|---|---|---|---|
| Pfropfpolymerisat | 65% | 55% | 70% | 65% |
| Titandioxid | 29% | 38% | 25% | 15% |
| Farbpigment^{*)} | 6% | 8% | 5% | 6% |
| Kaolin | | | | 15% |

| | | | | |
|---|---|---|---|---|
| *) 1a) Sicovit Rot, 1b) Sicovit Gelb, 1c) Indigotinlack, 1 d) Brilliantblau; Formulierungen Nr. 2-4: Sicovit Rot | | | | |

Teilchengrößenverteilung (Dichteverteilung) Formulierung Nr. 1a): d(01) 684 µm, d (05) 927 µm und d(09) 1238 µm, Verteilungsbreite 0,591 , gemessen mit einem Camsizer der Firma Retsch Technology GmbH.

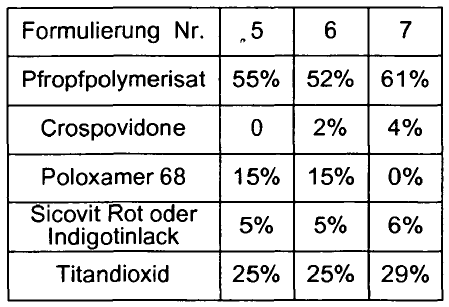

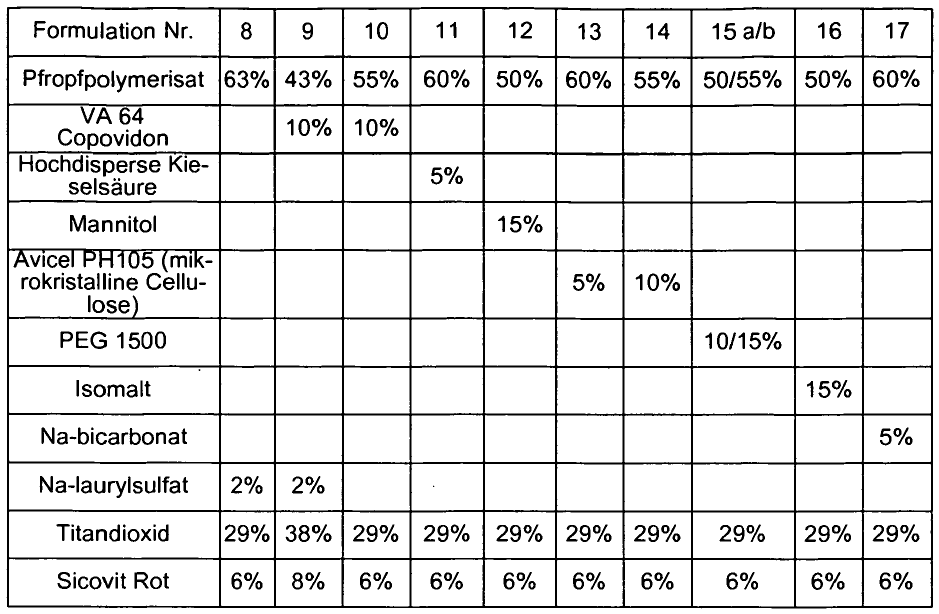

Die Extrudate und Wasser wurden bei einem Mischungsverhältnis von 20 Gew.-% Extrudat und 80 Gew.-% wasser vermischt. Innerhalb von 40 min waren die Extrudate vollständig dispergiert.

### Bestimmung der Staubzahl:

Die optische Konzentration des staubenden Anteils wurde durch Dispergierung in Luft nach freiem Fall in einem Fallrohr und Aufprall auf den Behälterboden bestimmt. 30 g einer Probe werden durch Öffnen einer Klappe in das Fallrohr eingebracht. Die Staubdichte wird durch Messung der Lichtschwächung eines Laserstrahls (Wellenlänge 670 nm) durch die entstehende Staubwolke bestimmt, wobei die Extinktion 0.5 s nach Öffnung der Klappe (Maximalwert) und 30 s nach Öffnung der Klappe bestimmt wird. Die Addition von Maximalwert und 30s-Wert ergibt die Staubzahl.

Die erfindungsgemäßen Extrudate weisen Staubzahlen von 3.8 (jeweils als Mittelwert von 3 Proben) auf. Zum Vergleich wurde der Staubwert eines Filmüberzugsmittels nach Bsp. 2 der WO 06/002808 ermittelt. Dieses Filmüberzugsmittel wurde wie in diesem Beispiel beschrieben durch Sprühtrocknung einer wässrigen Dispersion aus 61 % Pfropfpolymer, 7 % VA 64, 16 % Kaolin, 14% Titandioxid und 2% Na-laurylsulfat erhalten. Der Staubwert betrug 22.3.

### Bestimmung der elektrostatischen Aufladung:

Eine Polyethylentüte (25 x40 cm) wurde 1 min durch Reibung elektrostatisch aufgeladen, tariert und 40 g Extrudate eingewogen. Danach wurde die Einwaage ausgeleeert und die Tüte zurückgewogen. Im Mittel verbleiben 0.24 Gew.-%, bezogen auf die Einwaage, an Extrudat in der Tüte.
Zum Vergleich wurde wiederum das oben genannte Filmüberzugsmittel nach dem Stand der Technik untersucht. Dabei verblieben 3 Gew.-% der Einwaage in der Tüte.

## Patentansprüche

1. Feste teilchenförmige Filmüberzugsmittel, bestehend aus
a) 40 - 90 Gew.-%, bevorzugt 40 bis 70 Gew.-% eines Polyvinylalkohol-Polyether-Pfropfcopolymeren (Komponente A),
b) 10 bis 60 Gew.-%, bevorzugt 20 bis 45 Gew.-% eines bunten oder unbunten farbgebenden Mittels(Komponente B)
c) 0 bis 30 Gew.-% weiteren üblichen Hilfsstoffen (Komponenten C),
wobei sich die Menge der Komponenten A bis C zu 100 Gew.-% addiert, die durch Extrusion einer plastischen Masse der Komponenten A) bis C) bei erhöhter Temperatur der plastischen Mischung von 50 bis 160 °C und unter Einwirkung von Scherkräften und anschließende Formgebung erhalten werden.

2. Filmüberzugsmittel nach Anspruch 1, enthaltend als Komponente A ein Pfropfpolymer mit einem Massenverhältnis der Molekülteile Polyvinylalkohol zu Polyethylenglykol von 75:25, bei einem Molekulargewicht des Polyethylenglykols von 6000 Dalton.

3. Filmüberzugsmittel nach einem der Ansprüche 1 oder 2, enthaltend als Komponente B anorganische oder organische Pigmente und Farbstoffe oder Mischungen davon.

4. Filmüberzugsmittel nach einem der Ansprüche 1 bis 3, enthaltend als unbuntes farbgebendes Mittel ein Weißpigment.

5. Filmüberzugsmittel nach einem der Ansprüche 1 bis 4, enthaltend als Komponeten C Weichmacher, Tenside, wasserlösliche Farbstoffe, Antiklebemittel, polymere Bindemittel, Füllstoffe, Quellmittel, Glanzverstärker, Schaumverhinderer, Schutzkolloide, Puffersubstanzen, pH-regulierenden Substanzen, oder Haftvermittler.

6. Filmüberzugsmittel nach einem der Ansprüche 1 bis 5, enthaltend als Komponente B organische Farblacke.

7. Filmüberzugsmittel nach einem der Ansprüche 1 bis 6, enthaltend als Komponente B Eisenoxid, Titiandioxid, Kaolin. Talkum oder Mischungen davon.

8. Filmüberzugsmittel nach einem der Ansprüche 1 bis 7, enthaltend als Komponente C) Bindemittel aus der Gruppe der Homo- und Copolymere des N-Vinylpyrrolidons.

9. Filmüberzugsmittel nach einem der Ansprüche 1 bis 8, enthaltend als Komponente C einen Weichmacher ausgewählt aus der Gruppe bestehend aus Propylenglykol, Polyethylenglykolen, Polypropylenglykolen und Polyethylengykol-Polypropylenglykol-Blockcopolymeren..

10. Filmüberzugsmittel nach einem der Ansprüche 1 bis 9, enthaltend als Komponente C eine Füllstoff aus der Gruppe der Zucker oder der Zuckeralkohole.

11. Filmüberzugsmittel nach einem der Ansprüche 1 bis 10, enthaltend mikrokristalline Cellulose als Komponente C.

12. Filmüberzugsmittel nach einem der Ansprüche 1 bis 11, enthaltend als Komponente C Natriumlaurylsulfat.

13. Filmüberzugsmittel nach einem der Ansprüche 1 bis 12, enthaltend als Komponente C eine hochdisperse Kieselsäure.

14. Filmüberzugsmittel nach einem der Ansprüche 1 bis 13, erhältlich unter Verwendung eines Porenbildners.

15. Filmüberzugsmittel nach einem der Ansprüche 1 bis 14, erhältlich unter Verwendung einer Verbindung ausgewählt aus der Gruppe bestehend aus Carbonaten und Hydrogencarbonaten der Alkali- oder der Erdalkalimetalle und Zitronensäure als Porenbilder.

16. Filmüberzugsmittel nach einem der Ansprüche 1 bis 15, mit mittleren Teilchengrößen zwischen 500 und 1500 µm.

17. Verfahren zur Herstellung eines Filmüberzugsmittels gemäß einem der Ansprüche 1 bis 16, **dadurch gekennzeichnet, dass** die Komponenten A) bis C) unter Einwirkung von Scherkräften erhöhter Temperatur der plastischen Mischung von 50 bis 160 °C zu einer plastischen Masse verarbeitet, extrudiert und das Extrudat einer Formgebung zu scheibchenartigen Teilchen unterzogen wird.

18. Verfahren nach Anspruch 17, **dadurch gekennzeichnet, dass** die Herstellung der plastischen Masse in Gegenwart von Wasser erfolgt.

## Claims

1. A solid particulate film-coating composition consisting of
a) 40-90% by weight, preferably 40 to 70% by weight, of a polyvinyl alcohol-polyether graft copolymer (component A),
b) 10 to 60% by weight, preferably 20 to 45% by weight, of a chromatic or achromatic colorant (component B)
c) 0 to 30% by weight of further conventional auxiliaries (components C)
where the amount of components A to C adds up to 100% by weight, which are obtained by extrusion of a melt of components A) to C) at elevated temperature of the plastic mixture of from 50 to 160°C and by the action of shear forces and subsequent shaping.

2. The film-coating composition according to claim 1, comprising as component A a graft copolymer having a mass ratio of the polyvinyl alcohol to polyethylene glycol moieties of 75:25, with a molecular weight of the polyethylene glycol of 6000 daltons.

3. The film-coating composition according to either of claims 1 or 2, comprising as component B inorganic or organic pigments and dyes or mixtures thereof.

4. The film-coating composition according to any of claims 1 to 3, comprising as achromatic colorant a white pigment.

5. The film-coating composition according to any of claims 1 to 4, comprising as components C plasticizers, surfactants, water-soluble dyes, non-stick agents, polymeric binders, fillers, swelling agents, gloss improvers, antifoams, protective colloids, buffer substances, pH-regulating substances or bonding agents.

6. The film-coating composition according to any of claims 1 to 5, comprising as component B organic lakes.

7. The film-coating composition according to any of claims 1 to 6, comprising as component B iron oxide, titanium dioxide, kaolin, talc or mixtures thereof.

8. The film-coating composition according to any of claims 1 to 7, comprising as component C) binders from the group of homo- and copolymers of N-vinylpyrrolidone.

9. The film-coating composition according to any of claims 1 to 8, comprising as component C a plasticizer selected from the group consisting of propylene glycol, polyethylene glycols, polypropylene glycols and polyethylene glycol-polypropylene glycol block copolymers.

10. The film-coating composition according to any of claims 1 to 9, comprising as component C a filler from the group of sugars or of sugar alcohols.

11. The film-coating composition according to any of claims 1 to 10, comprising microcrystalline cellulose as component C.

12. The film-coating composition according to any of claims 1 to 11, comprising as component C sodium lauryl sulfate.

13. The film-coating composition according to any of claims 1 to 12, comprising as component C a colloidal silicon dioxide.

14. The film-coating composition according to any of claims 1 to 13, obtainable by use of a pore former.

15. The film-coating composition according to any of claims 1 to 14, obtainable via use of a compound selected from the group consisting of carbonates and bicarbonates of the alkali metals or alkaline earth metals and citric acid as pore former.

16. The film-coating composition according to any of claims 1 to 15, having average particle sizes of between 500 and 1500 µm.

17. A process for producing a film-coating composition according to any of claims 1 to 16, which comprises components A) to C) being processed by the action of shear forces at elevated temperature of the plastic mixture of from 50 to 160°C to a melt and extruded, and the extrudate being subjected to a shaping to disc-like particles.

18. The process according to claim 17, wherein the production of the melt takes place in the presence of water.

## Revendications

1. Agents de revêtement sous forme de film, particulaires, solides, constitués par
a) 40-90% en poids, de préférence 40 à 70% en poids d'un copolymère greffé de poly(alcool vinylique)-polyéther (Composant A),
b) 10 à 60% en poids, de préférence 20 à 45% en poids d'un agent coloré ou non coloré, conférant une couleur (Composant B)
c) 0 à 30% en poids d'autres adjuvants usuels (Composant C),
la somme des quantités des composants A à C valant 100% en poids, formés par extrusion d'une masse plastique des composants A) à C) à une température augmentée du mélange plastique de 50 à 160°C et sous l'effet de forces de cisaillement et façonnage consécutif.

2. Agents de revêtement sous forme de film selon la revendication 1, contenant, comme composant A, un polymère greffé présentant un rapport massique des parties moléculaires poly(alcool vinylique) à polyéthylèneglycol de 75:25, à un poids moléculaire du polyéthylèneglycol de 6000 Daltons.

3. Agents de revêtement sous forme de film selon l'une quelconque des revendications 1 ou 2, contenant comme composant B des pigments et des colorants inorganiques ou organiques ou leurs mélanges.

4. Agents de revêtement sous forme de film selon l'une quelconque des revendications 1 à 3, contenant comme agent non coloré conférant une couleur un pigment blanc.

5. Agents de revêtement sous forme de film selon l'une quelconque des revendications 1 à 4, contenant comme composant C des plastifiants, des agents tensioactifs, des solides solubles dans l'eau, des agents antiadhésifs, des liants polymères, des charges, des agents de gonflement, des agents de renforcement de la brillance, des antimousses, des colloïdes de protection, des substances tampon, des substances de régularisation du pH ou des promoteurs d'adhérence.

6. Agents de revêtement sous forme de film selon l'une quelconque des revendications 1 à 5, contenant, comme composant B, des laques colorées organiques.

7. Agents de revêtement sous forme de film selon l'une quelconque des revendications 1 à 6, contenant comme composant B de l'oxyde de fer, du dioxyde de titane, du kaolin, du talc ou leurs mélanges.

8. Agents de revêtement sous forme de film selon l'une quelconque des revendications 1 à 7, contenant comme composant C) des liants du groupe des homopolymères et des copolymères de la N-vinylpyrrolidone.

9. Agents de revêtement sous forme de film selon l'une quelconque des revendications 1 à 8, contenant comme composant C un plastifiant choisi dans le groupe constitué par le propylèneglycol, les polyéthylèneglycols, les polypropylèneglycols et les copolymères à blocs de polyéthylèneglycol-polypropylèneglycol.

10. Agents de revêtement sous forme de film selon l'une quelconque des revendications 1 à 9, contenant, comme composant C, une charge du groupe des sucres ou des alcools de sucre.

11. Agents de revêtement sous forme de film selon l'une quelconque des revendications 1 à 10, contenant, comme composant C, de la cellulose cristalline.

12. Agents de revêtement sous forme de film selon l'une quelconque des revendications 1 à 11, contenant, comme composant C, du laurylsulfate de sodium.

13. Agents de revêtement sous forme de film selon l'une quelconque des revendications 1 à 12, contenant, comme composant C, une silice hautement dispersée.

14. Agents de revêtement sous forme de film selon l'une quelconque des revendications 1 à 13, pouvant être obtenus avec utilisation d'un agent porogène.

15. Agents de revêtement sous forme de film selon l'une quelconque des revendications 1 à 14, pouvant être obtenus avec utilisation d'un composé, choisi dans le groupe constitué par les carbonates et les hydrogénocarbonates des métaux alcalins et alcalino-terreux et l'acide citrique comme agent porogène.

16. Agents de revêtement sous forme de film selon l'une quelconque des revendications 1 à 15, présentant des grosseurs moyennes de particule entre 500 et 1500 µm.

17. Procédé pour la préparation d'un agent de revêtement sous forme de film selon l'une quelconque des revendications 1 et 16, **caractérisé en ce que** les composants A) à C) sont transformés en masse plastique sous l'effet de forces de cisaillement, à une température augmentée du mélange plastique de 50 à 160°C, extrudés et le produit extrudé est soumis à un façonnage en particules en forme de disque.

18. Procédé selon la revendication 17, **caractérisé en ce que** la préparation de la masse plastique a lieu en présence d'eau.
